(19) Europäisches Patentamt
European Patent Office
Office européen des brevets

(11) EP 0 543 303 B1

(12) **EUROPÄISCHE PATENTSCHRIFT**

(45) Veröffentlichungstag und Bekanntmachung des Hinweises auf die Patenterteilung:
**12.02.1997 Patentblatt 1997/07**

(51) Int. Cl.$^6$: **C08F 283/06**, A61L 15/00, C08J 3/24

(21) Anmeldenummer: **92119512.9**

(22) Anmeldetag: **14.11.1992**

(54) **Hydrophile, hochquellfähige Hydrogele**

Hydrophilic hydrogels having a high swelling capacity

Hydrogels hydrophiles à forte capacité de gonflement

(84) Benannte Vertragsstaaten:
**AT BE CH DE DK ES FR GB GR IT LI NL SE**

(30) Priorität: **22.11.1991 DE 4138408**

(43) Veröffentlichungstag der Anmeldung:
**26.05.1993 Patentblatt 1993/21**

(73) Patentinhaber: **HOECHST AKTIENGESELLSCHAFT**
**65926 Frankfurt am Main (DE)**

(72) Erfinder:
- **Engelhardt, Friedrich, Dr.**
  **W-6000 Frankfurt am Main 60 (DE)**
- **Riegel, Ulrich**
  **W-6000 Frankfurt am Main 60 (DE)**
- **Kleiner, Hanss-Jerg, Dr.**
  **W-6242 Kronberg 2 (DE)**
- **Funk, Rüdiger, Dr.**
  **W-6200 Wiesbaden-Naurod (DE)**
- **Ebert, Gerlinde, Dr.**
  **W-6072 Dreieich/Offenthal (DE)**

(56) Entgegenhaltungen:
**EP-A- 0 481 370**    **DE-A- 3 911 443**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.13/3.4

## Beschreibung

Die vorliegende Erfindung betrifft hydrophile, hochquellfähige Hydrogela, die mit einer Phosphonsäurediglycidylester-haltigen Vernetzermischung oberflächenmodifiziert sind, Verfahren zu ihrer Herstellung, ihre Verwendung sowie Phosphonsäurediglycidylester-haltige Vernetzermischungen.

Hydrophile Hydrogele, die durch Polymerisation olefinisch ungesättigter Säuren, wie beispielsweise Acrylsäure, Methacrylsäure, Acrylamidopropansulfonsäure usw., in Gegenwart geringer Mengen mehrfach olefinisch ungesättigter Verbindungen erhalten werden können, sind bereits bekannt und beispielsweise beschrieben in US 4,057,521, US 4,062,817, US 4,525,527, US 4,286,082, US 4,340,706 und US 4,295,987.

Weiterhin sind auch hydrophile Hydrogele bekannt, die durch Pfropfcopolymerisation olefinisch ungesättigter Säuren auf unterschiedliche Matrices, wie beispielsweise Polysaccharide, Polyalkylenoxide sowie deren Derivate, zugänglich sind (z.B. US 5,011,892, US 4,076,663 und US 4,931,497).

Die genannten Hydrogele zeichnen sich durch ein hohes Aufnahmevermögen für Wasser und wäßrige Lösungen aus und finden daher bevorzugt Anwendung als Absorptionsmittel in Hygieneartikeln.

Es ist bereits bekannt, daß die Eigenschaften dieser Hydrogele durch eine Oberflächenbehandlung mit bestimmten Substanzen modifiziert werden können.

Beispielsweise sind Modifizierungen durch Behandlung mit kationischen Polymeren und Polyaminen (US 4,824,901), mit mehrwertigen Metallkationen (US 5,002,986 und US 4,771,105), mit anorganischen Zuschlägen (US 4,500,670), mit Alkoxysilylverbindungen (EP-A 415 183 und EP-A 195 406) sowie mit weiteren Substanzen wie Polyhydroxyverbindungen, Polyoxazolinen, Polyglycidylethern usw. (EP-A 372 981, EP-A 317 106, US 4,666,983 und US 4,734,478) bekannt.

Durch diese Modifizierungen können verbesserte Eigenschaften der Hydrogele hinsichtlich mechanischer Stabilität und Absorptionsgeschwindigkeit von Wasser erzielt werden.

Aufgabe vorliegender Erfindung ist es demgegenüber, Hydrogele mit verbesserten Eigenschaften hinsichtlich mechanischer Stabilität und Wasserrückhaltevermögen der gequollenen Partikel bereitzustellen.

Diese Aufgabe wird gelöst durch ein hydrophiles, hochquellfähiges Hydrogel auf Basis (co)polymerisierter hydrophiler Monomerer oder auf Basis von Pfropf(co)polymeren, dadurch gekennzeichnet, daß es oberflächenmodifiziert ist mit einer Mischung aus

A) einem oder mehreren Phosphonsäurediglycidylestern der allgemeinen Formel I

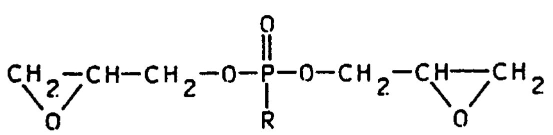

(I)

worin R Alkyl, Alkenyl oder Aryl, die gegebenenfalls substituiert sein können und

B) einer oder mehreren anderen reaktiven Verbindungen, die befähigt sind, mit den funktionellen Gruppen im Polymer zu reagieren.

Bevorzugt ist das erfindungsgemäße hydrophile, hochquellfähige Hydrogel oberflächenmodifiziert mit einer Mischung aus

A) einem oder mehreren Phosphonsäurediglycidylestern der allgemeinen Formel I

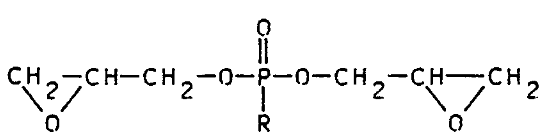

(I)

worin R ($C_1$-$C_{18}$)-Alkyl; ($C_3$-$C_8$)-Cycloalkyl; eine Gruppe der allgemeinen Formel II

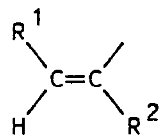

(II)

wobei $R^1$ und $R^2$ unabhängig voneinander für Wasserstoff oder $(C_1\text{-}C_4)$-Alkyl stehen; oder eine Gruppe der allgemeinen Formel III

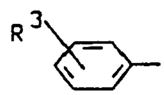

(III)

wobei $R^3$ für Wasserstoff, Halogen oder $(C_1\text{-}C_4)$-Alkyl steht, bedeutet und

B) einem oder mehreren (Poly-)glycidylethern und/oder einem oder mehreren Alkoxysilylverbindungen, und/oder einem oder mehreren Polyaziridinen und/oder einem oder mehreren Polyaminen und/oder einem oder mehreren Polyamidoaminen.

Für R, $R^1$, $R^2$ oder $R^3$ stehende Alkylgruppen können geradkettig oder verzweigt sein.

Für R stehendes $(C_1\text{-}C_{18})$-Alkyl bedeutet insbesondere Methyl, Ethyl, n-Propyl, i-Propyl, n-Butyl, i-Butyl, n-Pentyl, i-Pentyl, n-Hexyl, i-Hexyl, Decyl oder Stearyl, wobei aber $(C_1\text{-}C_3)$-Alkyl besonders bevorzugt ist.

Ein besonders bevorzugtes $(C_3\text{-}C_8)$-Cycloalkyl ist Cyclohexyl.

Für $R^1$ bzw. $R^2$ stehendes $(C_1\text{-}C_4)$-Alkyl ist besonders bevorzugt Methyl. $R^1$ und $R^2$ sind aber ganz besonders bevorzugt Wasserstoff.

Der Rest $R^3$ kann in 2-, 3- oder 4-Position bezogen auf die Kohlenstoff-Phosphor-Bindung stehen.

Für $R^3$ stehendes $(C_1\text{-}C_4)$-Alkyl bedeutet insbesondere Methyl, Ethyl, n-Propyl, i-Propyl, n-Butyl, i-Butyl oder tert.-Butyl. Für $R^3$ stehendes Halogen bedeutet insbesondere Fluor, Chlor, Brom oder Iod.

$R^3$ bedeutet besonders bevorzugt Methyl oder Chlor, ganz besonders bevorzugt Wasserstoff.

Besonders bevorzugte Verbindungen der allgemeinen Formel I sind Methylphosphonsäurediglycidylester, n-Propylphosphonsäurediglycidylester, Stearylphosphonsäurediglycidylester und Dekanphosphonsäurediglycidylester.

Bevorzugte (Poly-)glycidylether sind Monoethylenglykoldiglycidylether, Polyethylenglykoldiglycidylether, insbesondere Nonaethylenglykoldiglycidylether, Monopropylenglykoldiglycidylether und Polypropylenglykoldiglycidylether.

Bevorzugte Alkoxysilylverbindungen sind Aminoalkylalkoxysilane wie beispielsweise 3-Aminopropyltriethoxysilan, Trimethoxysilylpropyldiethylentriamin, N-Aminoethylaminopropyltrimethoxysilan und Aminoethylaminomethylphenethyltrimethoxysilan sowie Glycidylalkoxysilane wie beispielsweise 3-Glycidyloxypropyltrimethoxysilan.

Bevorzugte Polyaziridine sind Anlagerungsverbindungen von Aziridin an mehrfach ungesättigte Komponenten, beispielsweise Trimethylolpropantri[3-(1-aziridinyl)propionat].

Ein bevorzugtes Polyamin ist Polyethylenimin.

Bevorzugte Polyamidoamine sind Kondensate aus Adipinsäure und mehrwertigen Aminen, wie z.B. Diethylentriamin.

Das Verhältnis der Verbindung(en) der allgemeinen Formel I zu der bzw. den Verbindungen gemäß B) kann in weiten Grenzen variieren. Bevorzugt liegt der Anteil an Verbindung gemäß B) zwischen 0,5 und 90 Gew.%, besonders bevorzugt zwischen 1 und 60 Gew.%.

Werden mehrere Verbindungen der allgemeinen Formel I eingesetzt, so ist deren Gewichtsverhältnis völlig unkritisch. Analoges gilt, wenn mehrere Verbindungen gemäß B) eingesetzt werden.

In den erfindungsgemäßen Hydrogelen liegt der Anteil an Mischung aus Verbindung(en) der allgemeinen Formel I und Verbindung(en) gemäß B) bevorzugt bei 0,01 bis 10 Gew.%, besonders bevorzugt bei 0,05 bis 3 Gew.%.

Die den erfindungsgemäßen Hydrogelen zugrunde liegenden Polymere aus (co)polymerisierten hydrophilen Monomeren bzw. Pfropf(co)polymeren sind bekannt und beispielsweis in den oben zitierten Literaturstellen beschrieben.

Als hydrophile Monomere werden Acrylsäure, Methacrylsäure, Crotonsäure, 2-Acrylamido-2-methylpropansulfonsäure und -phosphonsäure, Vinylphosphonsäure, Vinylphosphonsäurehalbester, deren Salze, Acrylamid, N-Vinylamide oder Gemischen davon, wobei saure Gruppen ganz oder teilweise neutrali siert vorliegen können, eingesetzt.

Alternativ werden Produkte, die durch Pfropfpolymerisation von olefinisch ungesättigten Säuren auf Polysaccharide, Polyalkylenoxide oder deren Derivate zugänglich sind, eingesetzt.

Bevorzugt sind auch Pfropfpolymere auf Basis um Stärke, Cellulose oder Cellulosederivate, sowie die in US

4,931,497 und US 5,011,892 beschriebenen Pfropfpolymere.

Die den erfindungsgemäßen Hydrogelen zugrunde liegenden Polymere bzw. Pfropfpolymere können auch bereits mit geeigneten Vernetzern, wie beispielsweise Methylenbisacrylamid, Bisacrylamidoessigsäure, Alkenylphosphon- oder -phosphinsäureestern, Trimethylolpropantri(meth)acrylat oder Tetraallyloxyethan vernetzt sein.

Die erfindungsgemäßen Hydrogele weisen bevorzugt eine durchschnittliche Korngrößenverteilung von 20 bis 1000 µm, besonders bevorzugt 100 bis 1000 µm auf.

Bevorzugt sind darüber hinaus solche erfindungsgemäßen Hydrogele, bei denen der Masseanteil des Korngrößenbereichs 400 bis 1680 µm mehr als 50 % beträgt.

Die erfindungsgemäßen Hydrogele können dadurch hergestellt werden, daß die Polymere aus (co)polymerisierten hydrophilen Monomeren bzw. die Pfropf(co)polymeren in fester Form, bevorzugt in Pulverform mit einer Korngrössenverteilung zwischen 20 und 1000 µm, bevorzugt zwischen 100 und 850 µm, in einem Mischaggregat mit einer Mischung aus einer oder mehreren Verbindungen der allgemeinen Formel I und einer oder mehreren Verbindungen gemäß B) behandelt werden.

Geeignete Mischaggregate sind beispielsweise Patterson-Kelly-Mischer, DRAIS-Turbolenzmischer, Lödige-Mischer, Schneckenmischer, Tellermischer und Wirbelschichtmischer.

Das Verfahren wird bevorzugt im Temperaturbereich zwischen 0 und 200°C, bevorzugt 80 bis 180°C, ausgeführt. Es ist dabei bevorzugt, daß das zu modifizierende Polymer bzw. Pfropfpolymer vor Ausführung des Verfahrens auf eine Temperatur von 40 bis 100°C vorgewärmt wird. In einer bevorzugten Ausführungsform werden die Komponenten bei einer Temperatur zwischen 20 und 60°C in einem üblichen, heizbaren Mischaggregat gemischt und dann zur Beschleunigung der Reaktion im oberflächennahen Bereich auf eine Temperatur zwischen 80 und 200°C, bevorzugt 80 bis 150°C, erhitzt. In einer weiteren bevorzugten Ausführungsform erfolgt dieser Temperaturbehandlungsschritt in einem nachgeschalteten Trockner über einen Zeitraum von 10 Minuten bis 6 Stunden, wobei sowohl Spaltprodukte, welche bei der Reaktion entstehen können, sowie eventuell vorher zugesetzte Lösungsmittelanteile entfernt werden können.

Die Mischungen aus den Komponenten A) und B) können sowohl in Substanz als auch in Form von Lösungen eingesetzt werden.

Bevorzugte Lösungsmittel sind Wasser, sowie Alkohole, Ester, Ketone, Ether und Kohlenwasserstoffe sowie Mischungen dieser Komponenten mit Kochpunkten bis zu 200°C, bevorzugt bis 150°C.

Es ist auch möglich, die Polymeren aus (co)polymerisierten hydrophilen Monomeren bzw. die Pfropf(co)polymeren erst mit Komponente A und anschließend mit Komponente B zu modifizieren.

Gegenstand vorstehender Erfindung sind auch Vernetzermischungen bestehend aus

A) einem oder mehreren Phosphonsäurediglycidylestern der allgemeinen Formel I

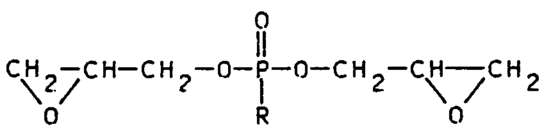

(I)

worin R Alkyl, Alkenyl oder Aryl, die gegebenenfalls substituiert sein können und

B) einer oder mehrerer anderer reaktiver Verbindungen, die befähigt sind, mit den funktionellen Gruppen des zu vernetzenden Polymers zu reagieren.

Bevorzugte Vernetzermischungen bestehen aus

A) einem oder mehreren Phosphonsäurediglycidylestern der allgemeinen Formel I

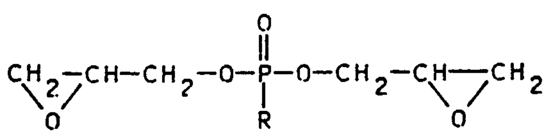

(I)

worin R ($C_1$-$C_{18}$)-Alkyl; ($C_3$-$C_8$)-Cycloalkyl; eine Gruppe der allgemeinen Formel II

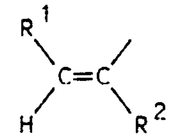

(II)

wobei $R^1$ und $R^2$ unabhängig voneinander für Wasserstoff oder $(C_1$-$C_4)$-Alkyl stehen; oder eine Gruppe der allgemeinen Formel III

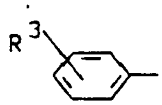

(III)

wobei $R^3$ für Wasserstoff, Halogen oder $(C_1$-$C_4)$-Alkyl steht, bedeutet und

B) einem oder mehreren (Poly-)glycidylethern und/oder einem oder mehreren Alkoxysilylverbindungen, und/oder einem oder mehreren Polyaziridinen und/oder einem oder mehreren Polyaminen und/oder einem oder mehreren Polyamidoaminen.

Weitere bevorzugte Ausführungsformen der erfindungsgemässen Vernetzermischungen hinsichtlich der Komponenten A) und B) entsprechen den bereits obengenannten bevorzugten Ausführungen.

Die erfindungsgemäßen Vernetzermischungen können durch einfaches Mischen der Komponenten erhalten werden.

Die Verbindungen der allgemeinen Formel I können nach bekannten Methoden hergestellt werden.

So ist beispielsweise in der US 2,856,369 die Umsetzung von Phosphonsäurediallylestern mit Persäuren zu den entsprechenden Phosphonsäurediglycidylestern der allgemeinen Formel I beschrieben, in Doklady Akad. SSSR, 155 (1964) 1137 die Umsetzung von Phosphonigsäuredichloriden mit Glycid in Gegenwart von Base, zu den entsprechenden Phosphonigsäurediglycidylestern, die ihrerseits mit Oxidationsmitteln wie $N_2O_4$ in Verbindungen der allgemeinen Formel I überführt werden können.

Bevorzugt werden die Verbindungen der allgemeinen Formel I hergestellt durch Umsetzung der entsprechenden Phosphonsäuredichloride mit Glycid in Gegenwart von Base. Die Base ist notwendig zum Abfangen des bei der Reaktion von Phosphonsäuredichlorid mit Glycid entstehenden HCl, das zu Neben- bzw. Folgereaktionen führt. In Zh. Obshch. Khim 116 (1984) 2404 wird als Base NaH empfohlen, häufiger genutzt werden jedoch stickstoffhaltige Basen. Hierzu zählen insbesondere tertiäre Amine wie Trimethylamin, Triethylamin, Tripropylamin oder Tributylamin. In US 2,856,369 wird auch die Verwendung von Pyridin empfohlen. Bevorzugt eingesetzt werden Trialkylamine, besonders bevorzugt Triethylamin.

Bevorzugte Lösemittel für diese Reaktionen sind Diethylether, Methyl-tert.-butylether, Benzol, Toluol oder Xylole, aber auch andere inerte Lösemittel sowie Mischungen verschiedener Lösemittel sind geeignet. Für technische Zwecke besonders geeignet sind Lösemittel wie Methyl-tert.-butylether, Tetrahydrofuran, Toluol oder Xylole sowie deren Mischungen, besonders bevorzugt wird Toluol eingesetzt.

Die Reaktanten sowie die erforderliche Base werden üblicherweise in stöchiometrischen Mengen eingesetzt, jedoch können auch Überschüsse an Base und/oder Glycid von Vorteil sein. Gründe für die jeweils bevorzugten Einsatzmengen können verfahrenstechnischer oder anwendungstechnischer Natur sein. Unter anwendungstechnische Gründe fallen u.a. Reinheitskriterien wie: Farbe, möglichst hohe Gehalte an Wirksubstanz, möglichst geringe Gehalte an Nebenprodukten, möglichst geringe Gehalte an Ausgangsverbindungen sowie möglichst geringe Gehalte an verseifbarem und/oder ionogenem Chlor. In letzterem Fall empfiehlt sich häufig ein geringer, 1-20 Mol-%, bevorzugt 1-5 Mol-% Überschuß an Amin.

Zur Reaktion wird üblicherweise eine Mischung aus Glycid und Base im verwendeten Lösemittel vorgelegt, und das Phosphonsäuredichlorid entweder in Substanz, oder in einem der beschriebenen Lösemittel gelöst zugetropft. Auch andere Verfahrensweisen sind möglich. Beispielsweise können das Amin und das Phosphonsäuredichlorid vorgelegt werden und das Glycid zugetropft werden. Auch kontinuierliche Verfahrensweisen sind möglich. Hierzu werden z.B. Ströme aus Glycid und Amin mit einem Strom des entsprechenden Phosphonsäuredichlorides zusammengeführt und derart zur Reaktion gebracht. Einer oder beide Ströme enhält dann das erforderliche Lösemittel, das nötig ist, um Verstopfungen der Rohrleitungen durch ausfallendes Aminhydrochlorid zu vermeiden.

Nach beendeter Reaktion wird üblicherweise das ausgefallene Aminhydrochlorid abgetrennt, beispielsweise durch

Filtration oder Zentrifugation. Wird ein lösemittelfreies Produkt benötigt, so wird nachfolgend das Lösemittel abdestilliert, gegebenenfalls unter vermindertem Druck. Eine weitere Reinigung des so erhaltenen Rohproduktes kann durch Destillation unter vermindertem Druck erfolgen, entweder aus der Blase, bevorzugt jedoch in kontinuierlicher Weise durch Destillation über einen Dünnschicht- oder Kurzwegverdampfer.

Die zum Einsatz kommenden Komponenten B sind bekannt und können nach dem Fachmann bekannten und in der einschlägigen Literatur beschriebenen Methoden hergestellt werden.

Zum Teil sind sie auch kommerziell erwerbbare Handelsprodukte.

Die erfindungsgemäßen hydrophilen, hochquellfähigen Hydrogele sind als Absorptionsmittel für Wasser und wässrige Lösungen in hervorragender Weise geeignet und können insbesondere bei der Herstellung von Hygieneartikeln wie Windeln, Binden, Tampons und anderen saugfähigen Produkten Verwendung finden. Von besonderem Vorteil ist dabei, daß die Polymerpartikel im gequollenen Zustand selbst unter mechanischer Belastung nicht zum Verkleben neigen.

Die Herstellung von Hygieneartikeln, insbesondere Windeln, unter Verwendung von Hydrogelen ist an sich dem Fachmann bekannt und in der Literatur beschrieben.

Beispiel 1

a) Herstellung des zu modifizierenden Polymers

In einem durch geschäumtes Kunststoffmaterial gut isolierten Polyethylengefäß mit einem Fassungsvermögen von 10 l werden 5240 g E-Wasser vorgelegt, 1505 g Natriumbicarbonat darin suspendiert und langsam 1980 g Acrylsäure so zudosiert, daß ein Überschäumen der Reaktionslösung vermieden wird, wobei sich diese auf eine Temperatur von ca. 5 bis 3°C abkühlt. Es wird nun eine Mischung aus 20 g Trimethylolpropantriacrylat in 16 g eines Polyglykolethers auf Basis eines synthetischen $C_{12}$-$C_{15}$-Oxoalkohols mit ca. 13 Ethylenoxideinheiten und 10 g eines Natrium-Diisooctylsulfosuccinates zugegeben. Bei einer Temperatur von 4°C werden die Initiatoren, ein Redoxsystem, bestehend aus 2,2 g 2,2'-Azobisamidinopropan-dihydrochlorid, gelöst in 20 g E-Wasser, 6 g Kaliumperoxodisulfat, gelöst in 170 g E-Wasser, sowie 0,4 g Ascorbinsäure, gelöst in 120 g E-Wasser nacheinander zugegeben und gut verrührt.

Die Reaktionslösung wird daraufhin ohne Rühren stehen gelassen, wobei durch einsetzende Polymerisation, in dessen Verlauf die Temperatur bis auf ca. 85°C ansteigt, ein festes Gel entsteht. Dieses wird anschließend mechanisch zerkleinert, bei Temperaturen über 80°C getrocknet, gemahlen und auf 100 bis 850 μm abgesiebt. Man erhält ein Produkt, gekennzeichnet durch folgende physikalische Daten, alle gemessen in NaCl 0,9 % :

| | |
|---|---|
| Absorption unter Druck (20 g/cm$^2$ - 1 Std.) | 9,4 g/g |
| Absorption (Teebeutel, Abtropftest-10/10 Min.) | 47 g/g |
| Zentrifugenretention (Teebeutel-10/3 Min.) | 36 g/g |
| Extrahierbare Anteile (16 Std.) | 17,4 % |

b) Oberflächenmodifizierung

In einem PETTERSON & KELLY-Mischer von 10 l Inhalt werden 6 kg Polymerpulver, hergestellt gemäß Beispiel 1a) vorgelegt. Unter Mischen wird im Verlauf von 7 Minuten eine Lösung aus 6 g Methylphosphonsäurediglycidylester, 6 g Nonaethylenglycoldiglycidylether und 228 g Wasser aufgedüst und 1 Minute nachgemischt. Das Produkt wird anschließend im Trockenschrank 30 Minuten bei 130°C nachgetempert. Es ist gekennzeichnet durch folgende physikalische Daten, alle gemessen in NaCl 0,9 % :

| | |
|---|---|
| Absorption unter Druck (20 g/cm$^2$ - 1 Std.) | 30,6 g/g |
| Absorption (Teebeutel, Abtropftest-10/10 Min.) | 46 g/g |
| Zentrifugenretention (Teebeutel-10/3 Min.) | 34 g/g |
| Extrahierbare Anteile (16 Std.) | 16,6 % |

Beispiel 2

a) Herstellung des zu modifizierenden Polymers

Unter adiabatischen Bedingungen werden in einem 5 l zylindrischen Weithalsreaktionskolben 3615 g auf 15°C abgekühltes E-Wasser vorgelegt, 490 g einer aufgekochten und ebenfalls auf 15°C abgekühlten Stärkelösung, bestehend aus 45 g Maisstärke und 445 g E-Wasser, 850 g Acrylsäure sowie 4,25 g Tetraallyloxyethan darin gelöst. Es wird Stickstoff in die Monomerlösung eingeleitet (ca. 21/Min.), um den Sauerstoffgehalt zu erniedrigen. Bei einem Gehalt von ca. 1,5 ppm $O_2$ werden 64 g einer 4 %igen wässrigen Lösung von 2,2'-Azobis(2-Amidinopropan)dihydrochlorid zugegeben, nach weiterem $N_2$-Einleiten und einem $O_2$-Gehalt von ca. 1,3 ppm werden 12 g einer 0,75 %igen $H_2O_2$-Lösung zugegeben und schließlich bei einem $O_2$-Gehalt von <1,0 ppm werden 12 g einer 0,15 %igen Ascorbinsäurelösung zugegeben. Durch einsetzende Polymerisation, in deren Verlauf die Temperatur bis auf ca. 65°C ansteigt, entsteht ein festes Gel, das anschließend mechanisch zerkleinert wird. 1000 g des zerkleinerten Gels werden mit 346 g Natronlauge 27 %ig versetzt (Neutralisationsgrad der Acrylsäure = 70 Mol-%), dreimal durchgeknetet, und anschließend bei Temperaturen über 150°C in dünner Schicht getrocknet, gemahlen und gegebenenfalls auf 50 bis 850 µm gesiebt.

Man erhält ein Produkt, gekennzeichnet durch folgende physikalische Daten, alle gemessen in NaCl 0,9 % :

| | |
|---|---|
| Absorption unter Druck (20 g/cm$^2$ - 1 Std.) | 15 g/g |
| Absorption (Teebeutel, Abtropftest-10/10 Min.) | 50 g/g |
| Zentrifugenretention (Teebeutel-10/3 Min.) | 36 g/g |
| Extrahierbare Anteile (16 Std.) | 7 % |

b) Oberflächenmodifizierung

In einem LÖDIGE-Pflugscharmischer von 100 l Inhalt werden 35 kg Polymerpulver mit einem Schüttgewicht von 0,46 g/cm$^3$, hergestellt gemäß Beispiel 2a), vorgelegt und auf 80°C angeheizt. Nach Erreichen der Produkttemperatur wird im Verlauf von 5 bis 10 Minuten eine Lösung von 87,5 g Propylphosphonsäurediglycidylester, 87,5 g eines glycidylorganofunktionellen Polydimethylsiloxans (TEGOMER ® E-SI 7235 / Handelsprodukt der TH. GOLDSCHMIDT AG) und 425 g Isopropanol und 50 g Wasser aufgedüst. Es wird auf 100°C Produkttemperatur angeheizt, um das Lösungsmittel wieder abzudestillieren, um anschließend direkt wieder abzukühlen.

Man erhält ein Produkt, das gekennzeichnet ist durch folgende physikalische Daten, alle gemessen in NaCl 0,9 % :

| | |
|---|---|
| Absorption unter Druck (20 g/cm$^2$ - 1 Std.) | 27 g/g |
| Absorption (Teebeutel, Abtropftest-10/10 Min.) | 61 g/g |
| Zentrifugenretention (Teebeutel-10/3 Min.) | 40 g/g |
| Extrahierbare Anteile (16 Std.) | 4,5 % |

Beispiel 3

a) Herstellung des zu modifizierenden Polymers

Gemäß US 4,931,497 werden in einem durch geschäumtes Kunststoffmaterial gut isolierten Polyethylengefäß mit einem Fassungsvermögen von 10 l 5310 g E-Wasser vorgelegt, 1219 g Natriumbicarbonat darin suspendiert und langsam 1972 g Acrylsäure so zudosiert, daß ein Überschäumen der Reaktionslösung vermieden wird, wobei sich diese auf eine Temperatur von ca. 5 bis 3°C abkühlt. Es wird nun eine Aufschlämmung aus 4 g Methylenbisacrylamid in 100 g Wasser sowie 24 g eines Umsetzungsproduktes aus 1 Mol Polyethylenglykol mit einem Durchschnittsmolekulargewicht von 300 und 1,98 Mol Maleinsäureanhydrid zugegeben. Bei einer Temperatur von 4°C werden die Initiatoren, ein Redoxsystem, bestehend aus 2,2 g 2,2'-Azobisamidinopropan-dihydrochlorid, gelöst in 20 g E-Wasser, 6 g Kaliumperoxodisulfat, gelöst in 175 g E-Wasser, sowie 0,4 g Ascorbinsäure, gelöst in 120 g E-Waser nacheinander zugegeben und gut verrührt. Die Reaktionslösung wird daraufhin ohne Rühren stehengelassen, wobei durch einsetzende Polymerisation, in dessen Verlauf die Temperatur bis auf ca. 85°C ansteigt, ein festes Gel entsteht.

Dieses wird anschließend mechanisch zerkleinert, bei Temperaturen über 80°C getrocknet, gemahlen und auf 100 bis 630 μm gesiebt.
Man erhält ein Produkt, gekennzeichnet durch folgende physikalische Daten, alle gemessen in NaCl 0,9 % :

| | |
|---|---|
| Absorption unter Druck (20 g/cm$^2$ - 1 Std.) | 8,5 g/g |
| Absorption (Teebeutel, Abtropftest-10/10 Min.) | 46 g/g |
| Zentrifugenretention (Teebeutel-10/3 Min.) | 32 g/g |
| Extrahierbare Anteile (16 Std.) | 6,5 % |

b) Oberflächenmodifizierung
In einem PETTERSON & KELLY-Mischer von 10 l Inhalt werden 6 kg Polymerpulver, hergestellt gemäß Beispiel 3a), vorgelegt. Unter Mischen wird im Verlauf von 5 Minuten eine Lösung aus 1,2 g n-Propylphosphonsäurediglycidylester, 10,8 g Monoethylenglykoldiglycidylether und 228 g Wasser aufgedüst und 1 Minute nachgemischt. Das Produkt wird anschließend im Trockenschrank 30 Minuten bei 130°C nachgetempert. Es ist gekennzeichnet durch folgende physikalische Daten, alle gemessen in NaCl 0,9 % :

| | |
|---|---|
| Absorption unter Druck (20 g/cm$^2$ - 1 Std.) | 24,9 g/g |
| Absorption (Teebeutel, Abtropftest-10/10 Min.) | 45 g/g |
| Zentrifugenretention (Teebeutel-10/3 Min.) | 38 g/g |
| Extrahierbare Anteile (16 Std.) | 6,8 % |

Beispiel 4

In einem 21-Polymerisationskolben werden 635 g Cyclohexan vorgelegt und unter Rühren auf 40 bis 45°C erwärmt, worauf die Zugabe von 3,5 g Ethylcellulose (Typ CN 200 der Fa. HERCULES, USA) erfolgt. Unter Einleiten eines schwachen $N_2$-Stromes wird auf Rückflußtemperatur erhitzt. Nach 25 Minuten Rückfluß wird mittels einer Dosierpumpe eine auf Raumtemperatur abgekühlte teilneutralisierte Acrylsäurelösung, bestehend aus 175 g Wasser, 230 g Acrylsäure, 258 g Kalilauge 50 %ig, abgemischt mit einer Lösung aus 20 g Wasser, 0,3 g Ethylendiamintetraessigsäure, 0,1 g $Na_2S_2O_8$ und 0,2 g 4,4'-Azobis-4-cyano-valeriansäure innerhalb von 90 Minuten zudosiert. Der Rückflußkühler wird nun gegen einen Wasserabscheider ausgetauscht und das Wasser wird azeotrop abdestilliert. Nach Beginn des azeotropen Abdestillierens des Wassers wird eine Emulsion, bestehend aus 15 g Cyclohexan, 0,5 g Wasser, 0,45 g Stearylphosphonsäurediglycidylester, 0,05 g Ethylenglykoldiglycidylester und 0,4 g eines Sorbitanmonolaurat, zugegeben. Es werden 260 g Wasser abdestilliert, das Lösungsmittel vom Polymer abfiltriert, 2 Stunden bei 105°C im Trockenschrank nachgetrocknet und gegebenenfalls von im Kornspektrum von der Norm abweichenden Polymerpartikeln abgesiebt. Man erhält ein Produkt, gekennzeichnet durch folgende physikalische Daten, alle gemessen mit der Kornfraktion 0,3 bis 0,4 mm in NaCl 0,9 % :

| | |
|---|---|
| Absorption unter Druck (20 g/cm$^2$ - 1 Std.) | 27 g/g |
| Absorption (Teebeutel, Abtropftest-10/10 Min.) | 46 g/g |
| Zentrifugenretention (Teebeutel-10/3 Min.) | 32 g/g |
| Extrahierbare Anteile (16 Std.) | 12 % |

Zur Charakterisierung der erfindungsgemäßen Hydrogele wurden von den folgenden Beispielen die Absorption unter Druckbelastung (AUL), die freie Absorption (FSC), die Zentrifugenretention (CR) und der Elastizitätsmodul G' gemessen.

Die Absorption unter Druckbelastung (20 g/cm$^2$) wird nach der in der EP 0 339 461, Seite 7, beschriebenen

Methode bestimmt: In einem Zylinder mit Siebboden gibt man die Einwaage an Hydrogel und belastet das Pulver mit einem Stempel, der einen Druck von 20 $g/cm^2$ ausübt. Der Zylinder wird anschließend auf einem Demand-Absorbency-Tester (DAT) gestellt, wo man den Superabsorber eine Stunde lang 0,9 %ige NaCl-Lösung saugen läßt.

Die freie Absorption und die Zentrifugenretention werden mit Hilfe der Teebeutelmethode bestimmt und als Mittelwert von zwei Messungen angegeben: Etwa 0,2 g Hydrogel werden in einen Teebeutel eingeschweißt und ür 10 Minuten in 0,9 %ige NaCl-Lösung getaucht. Zur Bestimmung der Absorption wird der Teebeutel nun diagonal für 10 Minuten aufgehängt und anschließend gewogen. Zur Bestimmung der Retention wird der Teebeutel nach dem Eintauchen in einer Schleuder (23 cm Durchmesser, 1400 Upm) 3 Minuten geschleudert und gewogen. Mit Hilfe eines Teebeutels ohne Hydrogel wird der Blindwert bestimmt:

$$\text{Absorption/Retention} = \frac{\text{Auswaage - Blindwert}}{\text{Einwaage}} \text{ (g/g)}$$

Der Elastizitätsmodul wird mit einem Carri-Med-Stress-Rheometer mit einer Platte-Platte-Konfiguration gemessen. Zur Bestimmung des Elastizitätsmoduls läßt man 1 g Hydrogel in 60 g 0,9 %iger NaCl-Lösung für 24 Stunden quellen und mißt anschließend an diesem gequollenen Gel den Speichermodul in Abhängigkeit von der Schubspannung bei einer Frequenz von 1 Hz. Der Plateauwert wird als Elastizitätsmodul G' angegeben.

Beispiels 5 - 32

a) Herstellung des zu modifizierenden Polymers

Man verfährt wie in Beispiel 2a angegeben mit der Ausnahme, daß statt 4,25 g nur 2,5 g Tetraallyloxyethan eingesetzt werden. Das getrocknete und gemahlene Produkt wird auf 100 - 850 µm abgesiebt. Die physikalischen Daten sind Tabelle 1 zu entnehmen.

b) Oberflächenmodifizierung

Je 100 g Pulver gemäß a) werden unter kräftigem Rühren mit je 10 g einer Vernetzerlösung, die die Komponenten A und B enthält, vermischt und anschließend für 2 Stunden in einem auf 120°C temperierten Ofen erhitzt. Nach dem Abkühlen werden die obengenannten Meßwerte bestimmt, die Tabelle 1 zu entnehmen sind.

Es werden folgende Abkürzungen verwendet:

IPA: Isopropylalkohol
A1: n-Propylphosphonsäurediglycidylester
A2: Decylphosphonsäurediglycidylester
A3: Phenylphosphonsäurediglycidylester
A4: Stearylphosphonsäurediglycidylester
B1: Monoethylenglykoldiglycidylether, epoxy equivalent (g/equ.) = 112
B2: Nonaethylenglykoldiglycidylether, epoxy equivalent (g/equ.) = 276
B3: Glycidylorg. funkt. Polydimethylsiloxan mit Kammstruktur, Epoxygruppen seitenständig, epoxy equivalent (g/equ.) = 500 - 600
B4: Glycidylorg. funkt. Polydimethylsiloxan mit Kammstruktur, Epoxygruppen seitenständig, epoxy equivalent (g/equ.) = 1000 - 1200
B5: a,w-glycidyl. funkt. Polydimethylsiloxan, Epoxygruppen endständig, epoxy equivalent (g/equ.) = 550 - 630
B6: a,w-glycidyl. funkt. Polydimethylsiloxan, Epoxygruppen endständig, epoxy equivalent (g/equ.) = 1100 - 1230

**Tabelle 1**

| Beispiel | Vernetzer Komponente A | Vernetzer Menge (Gew.%) | Komponente B | Menge (Gew.%) | Vernetzerlösung | AUL (g/g) | FSC (g/g) | CR (g/g) | FM $(N/m^2)$ |
|---|---|---|---|---|---|---|---|---|---|
| 5a | - | - | - | - | | 13,8 | 63 | 42 | 150 |
| Vergleich | - | - | - | - | IPA/$H_2O$ 50/50 | 13,9 | 62 | 42 | 158 |
| 5 | A1 | 0,25 | B1 | 0,25 | IPA/$H_2O$ 50/50 | 32,8 | 60 | 33 | 745 |
| 6 | A1 | 0,25 | B1 | 2,25 | IPA/$H_2O$ 50/50 | 31,8 | 61 | 34 | 710 |
| 7 | A1 | 1,00 | B1 | 1,00 | IPA/$H_2O$ 50/50 | 33,0 | 59 | 30 | 810 |
| 8 | A1 | 2,50 | B1 | 0,25 | IPA/$H_2O$ 50/50 | 32,2 | 60 | 31 | 850 |
| 9 | A1 | 2,50 | B1 | 2,50 | IPA/$H_2O$ 50/50 | 32,0 | 59 | 31 | 860 |
| 10 | A1 | 0,25 | B2 | 0,25 | IPA/$H_2O$ 50/50 | 32,4 | 62 | 33 | 680 |
| 11 | A1 | 0,25 | B2 | 2,25 | IPA/$H_2O$ 50/50 | 30,1 | 58 | 32 | 735 |
| 12 | A1 | 1,00 | B2 | 1,00 | IPA/$H_2O$ 50/50 | 29,3 | 60 | 32 | 675 |
| 13 | A1 | 2,50 | B2 | 0,25 | IPA/$H_2O$ 50/50 | 28,8 | 60 | 35 | 645 |
| 14 | A1 | 2,50 | B2 | 2,50 | IPA/$H_2O$ 50/50 | 31,7 | 57 | 34 | 710 |
| 12 | A2 | 1,00 | B2 | 1,00 | IPA/$H_2O$ 50/50 | 32,0 | 59 | 36 | 705 |
| 16 | A3 | 1,00 | B2 | 1,00 | IPA/$H_2O$ 50/50 | 31,8 | 58 | 35 | 720 |
| 17 | A4/A1 | 0,01/0,01 | B1 | 0,15 | IPA/$H_2O$ 50/50 | 26,4 | 59 | 36 | 480 |
| 18 | A1 | 0,25 | B3 | 0,25 | IPA/$H_2O$ 70/30 | 25,2 | 61 | 40 | 525 |
| 19 | A1 | 0,25 | B3 | 2,25 | IPA/$H_2O$ 85/15 | 15,1 | 60 | 40 | 405 |

**Tabelle 1**

| Beispiel | Vernetzer Kompo-nente A | Menge (Gew.%) | Kompo-nente B | Menge (Gew.%) | Vernetzerlösung | AUL (g/g) | FSC (g/g) | CR (g/g) | FM (N/m$^2$) |
|---|---|---|---|---|---|---|---|---|---|
| 20 | A1 | 2,50 | B3 | 2,50 | IPA/$H_2O$ 90/10 | 15,9 | 59 | 35 | 400 |
| 21 | A1 | 0,25 | B4 | 0,25 | IPA | 21,2 | 58 | 36 | 615 |
| 22 | A1 | 1,00 | B4 | 1,00 | IPA | 17,3 | 59 | 36 | 555 |
| 23 | A1 | 2,50 | B4 | 0,25 | IPA | 15,1 | 50 | 36 | 498 |
| 24 | A1 | 2,50 | B4 | 2,50 | IPA | 16,3 | 57 | 34 | 523 |
| 25 | A1 | 0,25 | B5 | 0,25 | IPA/$H_2O$ 90/10 | 21,2 | 58 | 33 | 640 |
| 26 | A1 | 0,25 | B5 | 2,25 | IPA/$H_2O$ 90/10 | 21,4 | 60 | 31 | 612 |
| 27 | A1 | 1,00 | B5 | 1,00 | IPA/$H_2O$ 90/10 | 15,5 | 47 | 37 | 535 |
| 28 | A1 | 2,50 | B5 | 2,50 | IPA/$H_2O$ 90/10 | 17,6 | 57 | 35 | 500 |
| 29 | A1 | 0,25 | B6 | 0,25 | IPA | 15,4 | 60 | 36 | 430 |
| 30 | A1 | 0,25 | B6 | 2,25 | IPA | 17,1 | 57 | 35 | 495 |
| 31 | A1 | 1,00 | B6 | 1,00 | IPA | 16,8 | 55 | 36 | 510 |
| 32 | A4 | 1,00 | B3/B5 | 2,00/7,00 | IPA | 26,3 | 48 | 32 | 920 |

Beispiele 33 - 67

Je 100 g Pulver gemäß Beispiel 5a werden unter kräftigem Rühren nacheinander mit je 5 g einer Vernetzerlösung

enthaltend Komponente A und je 5 g einer Vernetzerlösung, enthaltend Komponente B vermischt und anschließend für 2 Stunden in einem auf 120°C temperierten Ofen erhitzt. Nach dem Abkühlen werden die obengenannten Meßwerte bestimmt, die Tabelle 2 zu entnehmen sind.

Es wurden folgende zusätzliche Abkürzungen verwendet:

B7: 3-Glycidyloxypropyl-Trimethoxysilan
B8: (Aminoethylaminomethyl)phenylmethoxysilan
B9: 3-Aminopropyltriethoxysilan
B10: Trimethoxysilylpropyldiethylentriamin
B11: handelsübliches Polyamidoamin
B12: handelsübliches Polyaziridin
B13: handelsübliches Polyethylenimin

**Tabelle 2**

| Beispiel | Vernetzer Komponente A | Menge (Gew.%) | Komponente B | Menge (Gew.%) | Vernetzerlösung | AUL (g/g) | FSC (g/g) | CR (g/g) | EM ($N/m^2$) |
|---|---|---|---|---|---|---|---|---|---|
| 33 | A1 | 0,25 | B7 | 0,25 | IPA/$H_2O$ 50/50 | 33,2 | 62 | 33 | 810 |
| 34 | A1 | 0,25 | B7 | 2,25 | IPA/$H_2O$ 50/50 | 25,7 | 60 | 34 | 695 |
| 35 | A1 | 1,00 | B7 | 1,00 | IPA/$H_2O$ 50/50 | 30,6 | 60 | 34 | 731 |
| 36 | A1 | 2,50 | B7 | 0,25 | IPA/$H_2O$ 50/50 | 22,6 | 63 | 36 | 683 |
| 37 | A1 | 2,50 | B7 | 2,50 | IPA/$H_2O$ 50/50 | 21,3 | 59 | 36 | 680 |
| 38 | A1 | 0,25 | B8 | 0,25 | IPA/$H_2O$ 75/25 | 32,8 | 61 | 35 | 680 |
| 39 | A1 | 0,25 | B8 | 2,25 | IPA/$H_2O$ 75/25 | 31,2 | 58 | 34 | 735 |
| 40 | A1 | 1,00 | B8 | 1,00 | IPA/$H_2O$ 75/25 | 29,8 | 57 | 33 | 675 |
| 41 | A1 | 2,50 | B8 | 0,25 | IPA/$H_2O$ 75/25 | 29,3 | 51 | 28 | 645 |
| 42 | A1 | 2,50 | B8 | 2,50 | IPA/$H_2O$ 75/25 | 30,7 | 46 | 27 | 710 |
| 43 | A1 | 0,25 | B9 | 0,25 | IPA/$H_2O$ 75/25 | 32,7 | 60 | 32 | 722 |
| 44 | A1 | 0,25 | B9 | 2,25 | IPA/$H_2O$ 75/25 | 28,4 | 57 | 32 | 614 |
| 45 | A1 | 1,00 | B9 | 1,00 | IPA/$H_2O$ 75/25 | 30,4 | 54 | 29 | 654 |
| 46 | A1 | 2,50 | B9 | 0,25 | IPA/$H_2O$ 75/25 | 30,3 | 53 | 28 | 670 |
| 47 | A1 | 2,50 | B9 | 2,50 | IPA/$H_2O$ 75/25 | 30,4 | 52 | 28 | 655 |
| 48 | A1 | 0,25 | B10 | 0,25 | IPA/$H_2O$ 75/25 | 26 | 65 | 34 | 525 |
| 49 | A1 | 0,25 | B10 | 2,25 | IPA/$H_2O$ 75/25 | 19,6 | 64 | 34 | 512 |
| 50 | A1 | 1,00 | B10 | 1,00 | IPA/$H_2O$ 75/25 | 28,5 | 57 | 33 | 680 |
| 51 | A1 | 2,50 | B10 | 0,25 | IPA/$H_2O$ 75/25 | 30,5 | 56 | 31 | 653 |

**Tabelle 2**

| Beispiel | Vernetzer Kompo-nente A | Vernetzer Menge (Gew.%) | Vernetzer Kompo-nente B | Vernetzer Menge (Gew.%) | Vernetzerlösung | AUL (g/g) | FSC (g/g) | CR (g/g) | EM ($N/m^2$) |
|---|---|---|---|---|---|---|---|---|---|
| 52 | A1 | 2,50 | B10 | 2,50 | IPA/$H_2O$ 75/25 | 30,5 | 46 | 30 | 624 |
| 53 | A1 | 0,25 | B11 | 0,25 | IPA/$H_2O$ 75/25 | 31,5 | 54 | 31 | 615 |
| 54 | A1 | 0,25 | B11 | 2,25 | IPA/$H_2O$ 75/25 | 30,1 | 59 | 30 | 555 |
| 55 | A1 | 1,00 | B11 | 1,00 | IPA/$H_2O$ 75/25 | 29,8 | 57 | 29 | 500 |
| 56 | A1 | 2,50 | B11 | 0,25 | IPA/$H_2O$ 75/25 | 25,5 | 45 | 24 | 523 |
| 57 | A1 | 2,50 | B11 | 2,50 | IPA/$H_2O$ 75/25 | 26,9 | 48 | 24 | 540 |
| 58 | A1 | 0,25 | B12 | 0,25 | IPA/$H_2O$ 75/25 | 30,5 | 55 | 29 | 678 |
| 59 | A1 | 0,25 | B12 | 2,25 | IPA/$H_2O$ 75/25 | 28,2 | 58 | 30 | 610 |
| 60 | A1 | 1,00 | B12 | 1,00 | IPA/$H_2O$ 75/25 | 28,8 | 48 | 26 | 535 |
| 61 | A1 | 2,50 | B12 | 0,25 | IPA/$H_2O$ 75/25 | 28,3 | 44 | 25 | 500 |
| 62 | A1 | 2,50 | B12 | 2,50 | IPA/$H_2O$ 75/25 | 29,3 | 46 | 27 | 531 |
| 63 | A1 | 0,25 | B13 | 0,25 | IPA/$H_2O$ 75/25 | 31,8 | 57 | 34 | 778 |
| 64 | A1 | 0,25 | B13 | 2,25 | IPA/$H_2O$ 75/25 | 27,9 | 51 | 34 | 614 |
| 65 | A1 | 1,00 | B13 | 1,00 | IPA/$H_2O$ 75/25 | 29,7 | 46 | 30 | 675 |
| 66 | A1 | 2,50 | B13 | 0,25 | IPA/$H_2O$ 75/25 | 30,4 | 64 | 29 | 715 |
| 67 | A1 | 2,50 | B13 | 2,50 | IPA/$H_2O$ 75/25 | 27,8 | 55 | 26 | 625 |

## Patentansprüche

1. Hydrophiles, hochquellfähiges Hydrogel auf Basis (co)polymerisierter Acrylsäure, Methacrylsäure, Crotonsäure, 2-Acrylamido-2-methylpropansulfonsäure oder -phosphonsäure, Vinylphosphonsäure, Vinylphosphonsäurehalbester, deren Salze, Acrylamid, N-Vinylamide oder Gemischen davon, wobei saure Gruppen ganz oder teilweise neutralisiert vorliegen können, oder auf Basis von durch Pfropfcopolymerisation olefinisch ungesättigter Säuren auf Polysaccharide, Polyalkylenoxide sowie deren Derivate zugängliche Produkte, dadurch gekennzeichnet, daß es oberflächenmodifiziert ist mit einer Mischung aus

   A) einem oder mehreren Phosphonsäurediglycidylestern der allgemeinen Formel I

   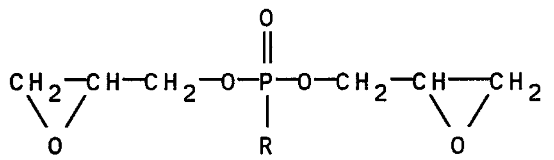

   (I)

   worin R Alkyl, Alkenyl oder Aryl, die gegebenenfalls substituiert sein können und
   B) einem oder mehrerern (Poly-)glycidylethern und/oder einem oder mehreren Alkoxysilylverbindungen und/oder einem oder mehreren Polyaziridinen und/oder einem oder mehreren Polyaminen und/oder einem oder mehreren Polyamidoaminen.

2. Hydrophiles, hochquellfähiges Hydrogel gemäß Anspruch 1, dadurch gekennzeichnet, daß es oberflächenmodifiziert ist mit einer Mischung aus

   A) einem oder mehreren Phosphonsäurediglycidylestern der allgemeinen Formel I

   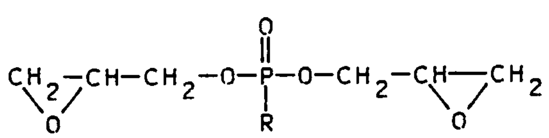

   (I)

   worin R $(C_1\text{-}C_{18})$-Alkyl; $(C_3\text{-}C_8)$-Cycloalkyl; eine Gruppe der allgemeinen Formel II

   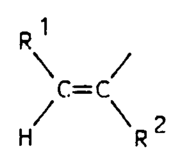

   (II)

   wobei $R^1$ und $R^2$ unabhängig voneinander für Wasserstoff oder $(C_1\text{-}C_4)$-Alkyl stehen; oder eine Gruppe der allgemeinen Formel III

   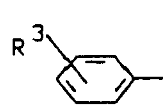

   (III)

   wobei $R^3$ für Wasserstoff, Halogen oder $(C_1\text{-}C_4)$-Alkyl steht, bedeutet und
   B) einem oder mehreren (Poly-)glycidylethern und/oder einem oder mehreren Alkoxysilylverbindungen, und/oder einem oder mehreren Polyaziridinen und/oder einem oder mehreren Polyaminen und/oder einem oder mehreren Polyamidoaminen.

3. Hydrophiles, hochquellfähiges Hydrogel gemäß Anspruch 1 und/oder 2, dadurch gekennzeichnet, daß in der Mischung aus Verbindung(en) der allgemeinen Formel I und Verbindung(en) gemäß B) der Anteil an B) 0,5 bis 90 Gew.%, besonders bevorzugt 1 bis 60 Gew.% beträgt.

4. Hydrophiles, hochquellfähiges Hydrogel gemäß einem oder mehreren der Ansprüche 1 bis 3, dadurch gekennzeichnet, daß der Anteil der Mischung aus Verbindung(en) der allgemeinen Formel I und Verbindung(en) gemäß B) 0,01 bis 10 Gew.%, besonders bevorzugt 0,05 bis 3 Gew.% beträgt.

5. Verwendung eines hydrophilen, hochquellfähigen Hydrogels gemäß einem oder mehreren der Ansprüche 1 bis 4 als Absorptionsmittel für Wasser und wässrige Lösungen.

6. Verwendung eines hydrophilen, hochquellfähigen Hydrogels gemäß einem oder mehreren der Ansprüche 1 bis 4 zur Herstellung von Hygieneartikeln.

7. Verfahren zur Herstellung eines hydrophilen, hochquellfähigen Hydrogels gemäß einem oder mehreren der Ansprüche 1 bis 4, dadurch gekennzeichnet, daß Polymere aus (co)polymerisierten hydrophilen Monomeren bzw. Pfropf(co)polymeren in fester Form, bevorzugt in Pulverform mit einer Korngrößenverteilung zwischen 20 und 1000 µm, bevorzugt zwischen 100 und 850 µm, in einem Mischaggregat mit einer Mischung aus einer oder mehreren Verbindungen der allgemeinen Formel I und einer oder mehreren Verbindungen gemäß B) behandelt werden.

8. Vernetzermischung zur Oberflächenmodifizierung von hydrophilen, hochquellfähigen Hydrogelen auf Basis (co)polymerisierter Acrylsäure, Methacrylsäure, Crotonsäure, 2-Acrylamido-2-methylpropansulfonsäure oder -phosphonsäure, Vinylphosphonsäure, Vinylphosphonsäurehalbester, deren Salze, Acrylamid, N-Vinylamide oder Gemischen davon, wobei saure Gruppen ganz oder teilweise neutralisiert vorliegen können, oder auf Basis von durch Pfropfcopolymerisation olefinisch ungesättigter Säuren auf Polysaccharide, Polyalkylenoxide sowie deren Derivate zugängliche Produkte bestehend aus

A) einem oder mehreren Phosphonsäurediglycidylestern der allgemeinen Formel I

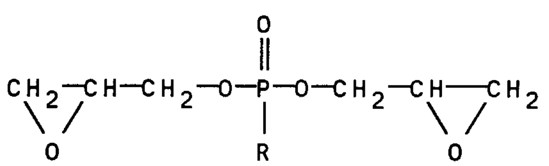

(I)

worin R Alkyl, Alkenyl oder Aryl, die gegebenenfalls substituiert sein können und

B) einem oder mehrerern (Poly-)glycidylethern und/oder einem oder mehreren Alkoxysilylverbindungen und/oder einem oder mehreren Polyaziridinen und/oder einem oder mehreren Polyaminen und/oder einem oder mehreren Polyamidoaminen.

9. Vernetzermischung gemäß Anspruch 8, bestehend aus

A) einem oder mehreren Phosphonsäurediglycidylestern der allgemeinen Formel I

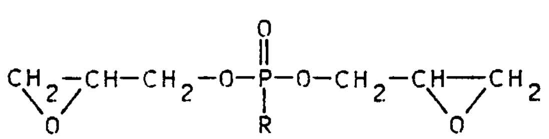

(I)

worin R ($C_1$-$C_{18}$)-Alkyl; ($C_3$-$C_8$)-Cycloalkyl; eine Gruppe der allgemeinen Formel II

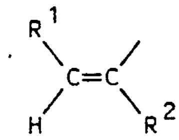

(II)

wobei $R^1$ und $R^2$ unabhängig voneinander für Wasserstoff oder ($C_1$-$C_4$)-Alkyl stehen; oder eine Gruppe der allgemeinen Formel III

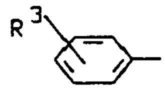

(III)

wobei $R^3$ für Wasserstoff, Halogen oder ($C_1$-$C_4$)-Alkyl steht, bedeutet und

B) einem oder mehreren (Poly-)glycidylethern und/oder einem oder mehreren Alkoxysilylverbindungen, und/oder einem oder mehreren Polyaziridinen und/oder einem oder mehreren Polyaminen und/oder einem oder mehreren Polyamidoaminen.

**10.** Verwendung einer Vernetzermischung gemäß Anspruch 8 und/oder 9 zur Oberflächenmodifizierung von Polymeren auf Basis (co)polymerisierter hydrophiler Monomere oder von Pfropf(co)polymeren.

## Claims

**1.** A hydrophilic hydrogel which has a high swelling capacity and is based on (co)polymerized acrylic acid, methacrylic acid, crotonic acid, 2-acrylamido-2-methylpropanesulfonic acid or -phosphonic acid, vinylphosphonic acid, a vinylphosphonic acid half-ester, a salt thereof, acrylamide, an N-vinylamide or a mixture thereof, it being possible for all or some acid groups to be present in neutralized form, or which is based on a product which is accessible by graft copolymerization of an olefinically unsaturated acid onto a polysaccharide, polyalkylene oxide or derivative thereof, wherein the hydrogel is modified on the surface with a mixture of

A) one or more phosphonic acid diglycidyl esters of the formula I

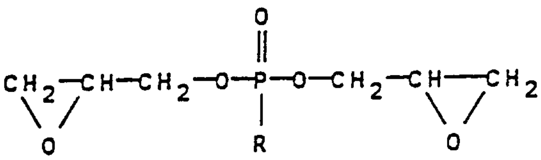

(I)

in which R is alkyl, alkenyl or aryl, which can optionally be substituted, and

B) one or more (poly)glycidyl ethers and/or one or more alkoxysilyl compounds and/or one or more polyaziridines and/or one or more polyamines and/or one or more polyamidoamines.

**2.** A hydrophilic hydrogel which has a high swelling capacity as claimed in claim 1, which is modified on the surface with a mixture of

A) one or more phosphonic acid diglycidyl esters of the formula I

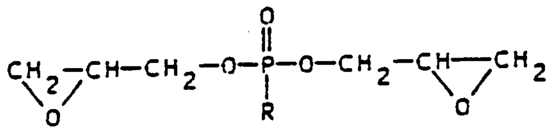

(I)

in which R is ($C_1$-$C_{18}$)-alkyl; ($C_3$-$C_8$)-cycloalkyl; a group of the formula II

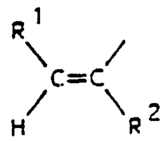

(II)

in which $R^1$ and $R^2$ independently of one another are hydrogen or ($C_1$-$C_4$)-alkyl; or a group of the formula III

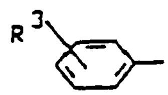

(III)

in which $R^3$ is hydrogen, halogen or ($C_1$-$C_4$)-alkyl and

B) one or more (poly)glycidyl ethers and/or one or more alkoxysilyl compounds and/or one or more polyaziridines and/or one or more polyamines and/or one or more polyamidoamines.

3. A hydrophilic hydrogel which has a high swelling capacity as claimed in claim 1 and/or 2, wherein, in the mixture of compound(s) of the formula I and compound(s) according to B), the content of B) is 0.5 to 90% by weight, particularly preferably 1 to 60% by weight.

4. A hydrophilic hydrogel which has a high swelling capacity as claimed in one or more of claims 1 to 3, wherein the content of the mixture of compound(s) of the formula I and compound(s) according to B) is 0.01 to 10% by weight, particularly preferably 0.05 to 3% by weight.

5. The use of a hydrophilic hydrogel which has a high swelling capacity as claimed in one or more of claims 1 to 4 as an absorbent for water and aqueous solutions.

6. The use of a hydrophilic hydrogel which has a high swelling capacity as claimed in one or more of claims 1 to 4 for the production of sanitary articles.

7. A process for the preparation of a hydrophilic hydrogel which has a high swelling capacity as claimed in one or more of claims 1 to 4, which comprises treating a polymer of a (co)polymerized hydrophilic monomer or graft (co)polymer in solid form, preferably in powder form having a particle size distribution of between 20 and 1000 μm, preferably between 100 and 850 μm, with a mixture of one or more compounds of the formula I and one or more compounds according to B) in a mixing unit.

8. A crosslinker mixture for modifying the surface of a hydrophilic hydrogel which has a high swelling capacity and is based on (co)polymerized acrylic acid, methacrylic acid, crotonic acid, 2-acrylamido-2-methylpropanesulfonic acid or -phosphonic acid, vinylphosphonic acid, a vinylphosphonic acid half-ester, a salt thereof, acrylamide, an N-vinylamide or a mixture thereof, it being possible for all or some acid groups to be present in neutralized form, or which is based on a product which is accessible by graft copolymerization of an olefinically unsaturated acid onto a polysaccharide, polyalkylene oxide or derivative thereof, comprising

A) one or more phosphonic acid diglycidyl esters of the formula I

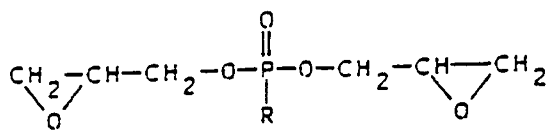 (I)

in which R is alkyl, alkenyl or aryl, which can optionally be substituted, and

B) one or more (poly)glycidyl ethers and/or one or more alkoxysilyl compounds and/or one or more polyaziridines and/or one or more polyamines and/or one or more polyamidoamines.

**9.** A crosslinker mixture as claimed in claim 8, comprising

A) one or more phosphonic acid diglycidyl esters of the formula I

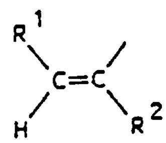 (II)

in which R is $(C_1\text{-}C_{18})$-alkyl; $(C_3\text{-}C_8)$-cycloalkyl; a group of the formula II

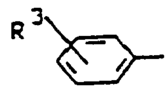 (III)

in which $R^1$ and $R^2$ independently of one another are hydrogen or $(C_1\text{-}C_4)$-alkyl; or a group of the formula III

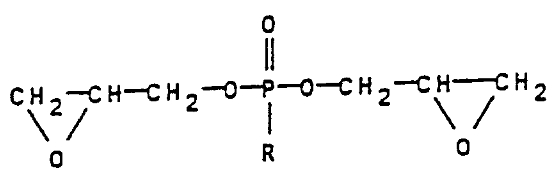 (I)

in which $R^3$ is hydrogen, halogen or $(C_1\text{-}C_4)$-alkyl and

B) one or more (poly)glycidyl ethers and/or one or more alkoxysilyl compounds and/or one or more polyaziridines and/or one or more polyamines and/or one or more polyamidoamines.

**10.** The use of a crosslinker mixture as claimed in claim 8 and/or 9 for modifying the surface of a polymer based on (co)polymerized hydrophilic monomers or of a graft (co)polymer.

## Revendications

**1.** Hydrogel hydrophile à grand pouvoir gonflant, à base des monomères (co)polymérisés de l'acide acrylique, l'acide méthacrylique, l'acide crotonique, l'acide 2-acrylamido-2-méthylpropanesulfonique ou -phosphonique, l'acide vinylphosphonique, l'hémi-ester de l'acide vinylphosphonique, leurs sels, l'acrylamide, les N-vinyl-acrylamides ou leurs mélanges, où les groupes acide peuvent être présents neutralisés en totalité ou en partie, ou encore à base de produits obtenus par copolymérisation par greffage d'acides à insaturation oléfinique sur des polysaccharides, poly(oxyalkylènes) ou leurs dérivés, caractérisé en ce qu'il est soumis à une modification superficielle avec un mélange

A) d'un ou plusieurs phosphonates de diglycidyle de formule générale I

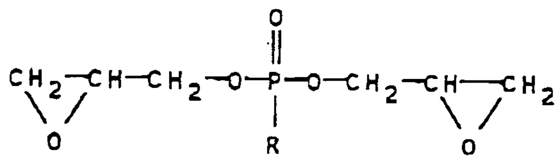

(I)

dans laquelle R est un groupe alkyle, alcényle ou aryle, qui éventuellement peuvent être substitués, et

B) d'un ou plusieurs (poly)glycidyléthers et/ou d'un ou plusieurs composés alcoxysilylés, et/ou d'une ou plusieurs polyaziridines, et/ou d'une ou plusieurs polyamines, et/ou d'une ou plusieurs polyamidoamines.

2. Hydrogel hydrophile à grand pouvoir gonflant selon la revendication 1, caractérisé en ce qu'il est modifié en surface avec un mélange

A) d'un ou plusieurs phosphonates de diglycidyle de formule générale I

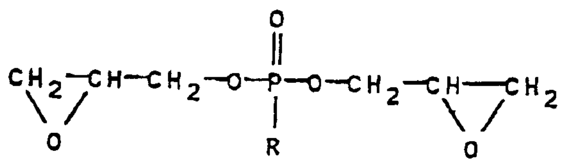

(I)

dans laquelle R est un radical alkyle en $C_1$-$C_{18}$ ; cycloalkyle en $C_3$-$C_8$ ; un groupe de formule générale II

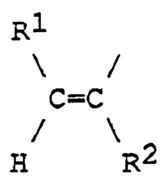

(II)

dans laquelle $R^1$ et $R^2$, indépendamment l'un de l'autre, sont chacun un hydrogène ou un radical alkyle en $C_1$-$C_4$ ; ou un groupe de formule générale III

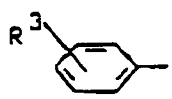

(III)

dans laquelle $R^3$ est un hydrogène, un halogène ou groupe alkyle en $C_1$-$C_4$, et

B) d'un ou plusieurs (poly)glycidyléthers et/ou d'un ou plusieurs composés alkoxysilylés, et/ou d'une ou plusieurs polyaziridines, et/ou d'une ou plusieurs polyamines, et/ou d'une ou plusieurs polyamidoamines.

3. Hydrogel hydrophile à grand pouvoir gonflant selon la revendication 1 et/ou 2, caractérisé en ce que, dans le mélange du ou des composés de formule générale I et du ou des composés selon B, la quantité de B est de 0,5 à 90 % en poids, et en particulier de 1 à 60 % en poids.

4. Hydrogel hydrophile à grand pouvoir gonflant selon l'une ou plusieurs revendications 1 à 3, caractérisé en ce que la quantité du mélange du ou des composés de formule générale I et du ou des composés selon B est de 0,01 à 10 % en poids, en particulier de 0,05 à 3 % en poids.

5. Utilisation d'un hydrogel hydrophile à grand pouvoir gonflant selon l'une ou plusieurs des revendications 1 à 4, comme absorbant de l'eau et de solutions aqueuses.

6. Utilisation d'un hydrogel hydrophile à grand pouvoir gonflant selon l'une ou plusieurs des revendications 1 à 4, pour

fabriquer des articles d'hygiène.

7. Procédé pour fabriquer un hydrogel hydrophile à grand pouvoir gonflant selon l'une ou plusieurs des revendications 1 à 4, caractérisé en ce qu'on traite des polymères constitués de monomères hydrophiles (co)polymérisés, ou encore des (co)polymères greffés sous forme solide, de préférence sous forme de poudre ayant une répartition granulométrique comprise entre 20 et 1000 µm et de préférence entre 100 et 850 µm, dans un équipement mélangeur, avec un mélange d'un ou plusieurs composés de formule générale I et d'un ou plusieurs composés selon B.

8. Mélange de réticulation pour la modification superficielle d'hydrogels hydrophiles à grand pouvoir gonflant, à base des monomères (co)polymérisés l'acide acrylique, l'acide méthacrylique, l'acide crotonique, l'acide 2-acrylamido-2-méthylpropanesulfonique ou -phosphonique, l'acide vinylphosphonique, l'hémi-ester de l'acide vinylphosphonique, leurs sels, l'acrylamide, les N-vinylacrylamides ou leurs mélanges, où les groupes acide peuvent être présents neutralisés en totalité ou en partie, ou encore à base de produits obtenus par copolymérisation par greffage d'acides à insaturation oléfinique sur des polysaccharides, poly(oxyalkylènes) ainsi que leurs dérivés, composé de

A) d'un ou plusieurs phosphonates de diglycidyle de formule générale I

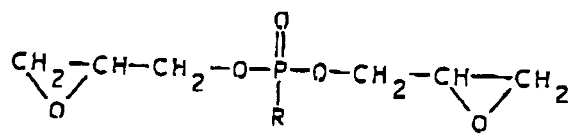 (I)

dans laquelle R est un groupe alkyle, alcényle ou aryle, qui éventuellement peuvent être substitués, et
B) d'un ou plusieurs (poly)glycidyléthers et/ou d'un ou plusieurs composés alkoxysilylés, et/ou d'une ou plusieurs polyaziridines, et/ou d'une ou plusieurs polyamines, et/ou d'une ou plusieurs polyamidoamines.

9. Mélange de réticulation selon la revendication 8, constitué

A) d'un ou plusieurs phosphonates de diglycidyle de formule générale I

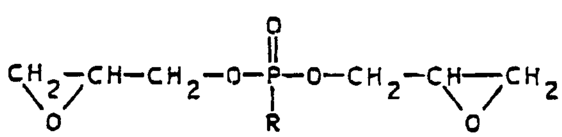 (I)

dans laquelle R est un radical alkyle en $C_1$-$C_{18}$ ; cycloalkyle en $C_3$-$C_8$ ; un groupe de formule générale II

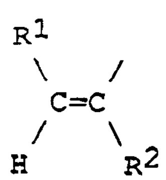 (II)

dans laquelle $R^1$ et $R^2$, indépendamment l'un de l'autre, sont chacun un hydrogène ou un radical alkyle en $C_1$-$C_4$ ; ou un groupe de formule générale III

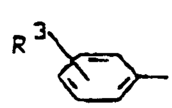 (III)

dans laquelle $R^3$ est un hydrogène, un halogène ou groupe alkyle en $C_1$-$C_4$, et
B) d'un ou plusieurs (poly)glycidyléthers et/ou d'un ou plusieurs composés alkoxysilylés, et/ou d'une ou plusieurs polyaziridines, et/ou d'une ou plusieurs polyamines, et/ou d'une ou plusieurs polyamidoamines.

10. Utilisation d'un mélange de réticulation selon la revendication 8 et/ou 9, pour la modification superficielle de polymères à base de monomères hydrophiles (co)polymérisés ou de (co)polymères greffés.